# DEMANDE DE BREVET EUROPEEN

(11) **EP 4 488 658 A1**
(43) Date de publication de la demande: **08.01.2025**
(21) Numéro de dépôt: 23183071.2
(22) Date de dépôt: 03.07.2023
(51) Int. Cl.: G01N 21/31, G01J 1/00, G01J 3/02, G01N 21/359

(54) **PROCEDE ET SYSTEME D'IDENTIFICATION DE MICROORGANISMES SOUS FORME DE COLONIE**

(71) Demandeur: BIOMÉRIEUX, 69280 Marcy L'Etoile (FR)
(72) Inventeur: LEROUX, Denis, 01600 TREVOUX (FR); LE MAUFF, Frédéric, 69007 LYON (FR)
(74) Mandataire: bioMérieux PI Groupement mandataires

(57) **Abrégé**

Il est décrit un procédé d'identification d'une souche microbienne sous forme de colonie, comportant l'acquisition d'un ensemble d'au moins trois canaux spectraux de la colonie compris dans le spectre visible et infrarouge; et la mise en oeuvre par ordinateur d'une prédiction de l'espèce en fonction des canaux spectraux acquis. Les trois canaux dudit ensemble les plus contributeurs à la performance de la prédiction sont choisis dans une première bande spectrale [730-780]nm et une deuxième bande spectrale [550-650]nm.

## Description

### DOMAINE DE L'INVENTION

L'invention a trait au domaine de l'analyse microbiologique, et en particulier de l'identification de bactéries, levures et moisissures ayant poussées sous forme de colonie sur un milieu nutritif, par exemple coulé dans une boite de Pétri, et de manière privilégiée un milieu nutritif non chromogène, non fluorogène et dépourvu de colorant.

De manière avantageuse, l'invention peut être mise en oeuvre à l'aide d'un dispositif portable de type smartphone ou tablette.

### ETAT DE LA TECHNIQUE

On sait que le spectre visible et proche infrarouge contient l'information nécessaire à l'identification de l'espèce d'une colonie bactérienne ayant poussé sur un milieu de culture, par exemple contenu dans une boite de Pétri, le milieu et la boite étant ci-après nommés ensemble "boite de Pétri".

A titre d'exemple, la Demanderesse atteint une précision d'identification de l'espèce formant un colonie par analyse au moyen d'apprentissage automatisé de l'image de cette colonie d'une dizaine de pixels prise par une caméra hyperspectrale à 240 canaux spectraux dans la gamme [390-900] nm supérieure à 95%, très souvent supérieure à 98%. On pourra se reporter aux documents Feng, Y.Z & al., "Invasive weed optimization for optimizing one-agar-for-all classification of bacterial colonies based on hyperspectral imaging. "? Sensors and Actuators B-Chemical (2018) 269: 264-270 et le document «Hyperspectral image analysis for rapid and accurate discrimination of bacterial infections: A benchmark study » de Arrigoni et al., Computers in Biology and Medicine 88 (2017)

Si la technologie hyperspectrale est aujourd'hui mature et peu onéreuse, une caméra de ce type coûtant une dizaine de milliers d'euros, elle reste cependant inaccessible dans de nombreuses situations, en particulier dans les pays en voie de développement. Outre le cout qui reste trop élevé, la technologie hyperspectrale est également trop peu mobile. Les caméras, encombrantes, restent à demeure dans les laboratoires, alors que les médecins microbiologistes sont itinérants pour atteindre les patients situés dans des endroits reculés du territoire.

Dans ce contexte, il existe un besoin d'identification pouvant être réalisée au moyen de dispositifs encore moins coûteux, mobiles, peu encombrants et réalisant seuls la totalité de l'identification, depuis la prise d'image jusqu'au au rendu de l'analyse, et ce avec des performances égales ou très proches de celles obtenues par l'utilisation d'une imagerie hyperspectrale ayant des centaines de canaux spectraux.

### EXPOSE DE L'INVENTION

Le but de la présente invention est de proposer un procédé performant d'identification de microorganismes sous forme de colonies par l'analyse d'un nombre limités de canaux dans le visible et proche infrarouge.

A cet effet, l'invention a pour objet un procédé d'identification d'une souche microbienne sous forme de colonie, comportant :
A. l'acquisition d'un ensemble d'au moins trois canaux spectraux de la colonie compris dans le spectre visible et infrarouge; et
B. la mise en oeuvre par ordinateur d'une prédiction de l'espèce en fonction des canaux spectraux acquis.

Selon l'invention, les trois canaux dudit ensemble les plus contributeurs à la performance de la prédiction sont choisis dans :
- une première bande spectrale [730-780]nm;
- une deuxième bande spectrale [550-650]nm.

L'invention a également pour objet un procédé d'identification d'un microorganisme susceptible d'être contenu dans un échantillon biologique d'un type prédéfini, comprenant:
A. l'acquisition d'au moins une image numérique multispectrale d'au plus dix canaux d'au moins une colonie microbienne ayant poussée sur un milieu de culture suite à l'étallement de l'échantillon sur ledit milieu;
B. la prédiction, mise en oeuvre par informatique, de l'espèce microbienne formant ladite colonie parmi un panel prédéterminé d'espèces microbienne en fonction de la ou des images multispectrales numériques acquises;

Selon l'invention, le panel d'espèces microbiennes, les filtres optiques et l'algorithme d'identification sont choisis de manière à ce que la spécificité de l'identification est supérieure ou égale à 90%, et de préférence supérieure ou égale à 94%;
- si le modèle de prédiction délivre l'identité d'une espèce
   ∘ alors ladite identité est confirmée sans mettre en oeuvre un autre procédé d'identification du microorganismes;
   ∘ sinon un autre procédé d'identification du microorganisme est mis en oeuvre.

L'invention a également pour objet un Dispositif d'identification d'une colonie microbienne parmi un panel prédéterminé d'espèces microbiennes responsables d'infections pathogéniques d'un type prédéterminé, notamment urinaire, comprenant:
A. une caméra numérique configurée pour acquérir une image numérique de ladite colonie;
B. et une unité de traitement informatique connectée à ladite caméra pour recevoir l'image numérique de la colonie;

Selon l'invention, la caméra numérique comprend:
A. 1. un capteur d'image monochromatique apte à capter une lumière incidente dans la gamme de longueurs d'onde [390-900] nm;
A.2.un objectif apte à former une image sur ledit capteur dans ladite gamme;
A.3.un ensemble d'au plus dix filtres passe-bande optiques centrés respectivement sur différentes longueurs d'onde de la gamme de longueurs d'onde [390-900] nm, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;
et les trois canaux dudit ensemble les plus contributeurs à la performance de la prédiction sont choisis dans :
- une première bande spectrale [730-780]nm;
- une deuxième bande spectrale [550-660]nm

L'invention a également pour objet un dispositif d'identification d'une colonie microbienne parmi un panel prédéterminé d'espèces microbiennes responsables d'infections pathogéniques d'un type prédéterminé, notamment urinaire, comprenant:
A. une caméra numérique configurée pour acquérir une image numérique de ladite colonie;
B. et une unité de traitement informatique connectée à ladite caméra pour recevoir l'image numérique de la colonie;

Selon l'invention, la caméra numérique comprend:
A.1. un capteur d'image monochromatique apte à capter une lumière incidente dans la gamme de longueurs d'onde [390-900] nm;
A.2. un objectif apte à former une image sur ledit capteur dans ladite gamme;
A.3. un ensemble d'au plus dix filtres passe-bande optiques centrés respectivement sur différentes longueurs d'onde de la gamme de longueurs d'onde [390-900] nm, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;

et l'unité de traitement informatique est configurée pour mettre en oeuvre un algorithme d'identification de l'espèce formant la colonie en fonction des images numériques,
et le panel d'espèces microbiennes correspond à une prévalence d'au moins 70% des infections pathologiques, et de préférence supérieure ou égale à 80%,
et le panel d'espèces microbiennes, les filtres optiques et l'algorithme d'identification sont choisis de manière à ce que la spécificité de l'identification est supérieure ou égale à 90%, et de préférence supérieure ou égale à 94%.

L'invention a également pour objet un Dispositif de caractérisation d'une colonie microbienne, comprenant:
A. une caméra numérique configurée pour acquérir une image numérique de ladite colonie;
B. et une unité de traitement informatique connectée à ladite caméra pour recevoir l'image numérique de la colonie;

selon l'invention, la caméra numérique comprend:
   A.1. un capteur d'image monochromatique apte à capter une lumière incidente dans la gamme de longueurs d'onde [390-900] nm;
   A.2.un objectif apte à former une image sur ledit capteur dans ladite gamme;
   A.3.un ensemble d'au plus dix filtres passe-bande optiques, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;
et l'ensemble d'au plus 10 filtres comprend:
   A.3.1.un premier filtre centré sur une longueur d'onde de la gamme [415-440]nm;
   A.3.2.des seconds filtres centrés respectivement sur différentes longueurs d'onde de la gamme de longueurs d'onde [390-900] nm
et l'unité de traitement informatique est configurée pour identifier:
   B. 1 .le gram de l'espèce formant la colonie en fonction de l'image numérique de ladite colonie acquise au moyen du premier filtre;
   B.2. l'identité de l'espèce formant la colonie en fonction des images numériques acquises à l'aide des seconds filtres.

### BREVE DESCRIPTION DES FIGURES

L'invention sera mieux comprise à la lecture de la description qui va suivre, donnée uniquement à titre d'exemple, et faire en relation avec les dessins annexés, dans lesquels des références identiques désignent des éléments identiques ou analogues, et dans lesquels
- la figure 1A est une schématique d'un système d'acquisition multi-spectrales et d'identification d'espèces microbiennes formant colonie selon l'invention;
- la **figure 2** est une vue éclatée de ce même système;
- la **figure 3** est une vue de dessus simplifiée d'une roue à filtre entrant dans la constitution de ce système;
- la **figure 4** est une vue schématique de la réponse fréquentielle d'un filtre passe-bande entrant dans la constitution de la roue à filtres;
- la **figure 5** illustre la réflectance à différents canaux spectraux pour différentes espèces, réflectance ici illustré par son diagramme en boite ("box-plot") au 95^{e} centile;
- la **figure 6** est la courbe du taux d'erreur de classification en fonction des canaux sélectionnés selon une approche "feed-forward";
- la **figure 7** illustre un arbre décisionnel pour la classification de 8 uropathogène en fonction de la réflectance à différents canaux spéctraux
- la **figure 8** illustre un autre arbre décisionnel pour la classification de 8 uropathogène en fonction de la réflectance à différents canaux spéctraux
- la **figure 9** est un organigramme de traitement d'échantillons urinaires se basant sur l'invention.

### DESCRIPTION DETAILLEE DE L'INVENTION

### A. Système d'identification multi-spectral selon l'invention

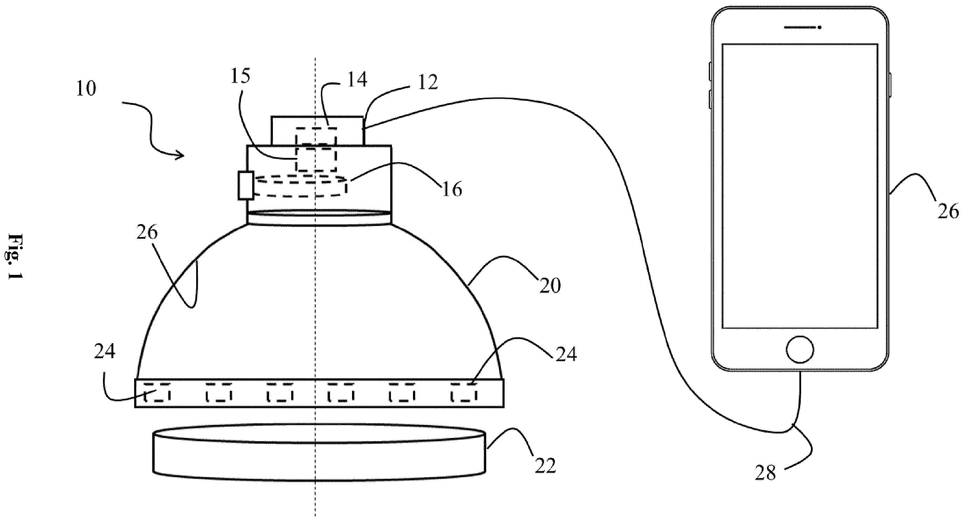

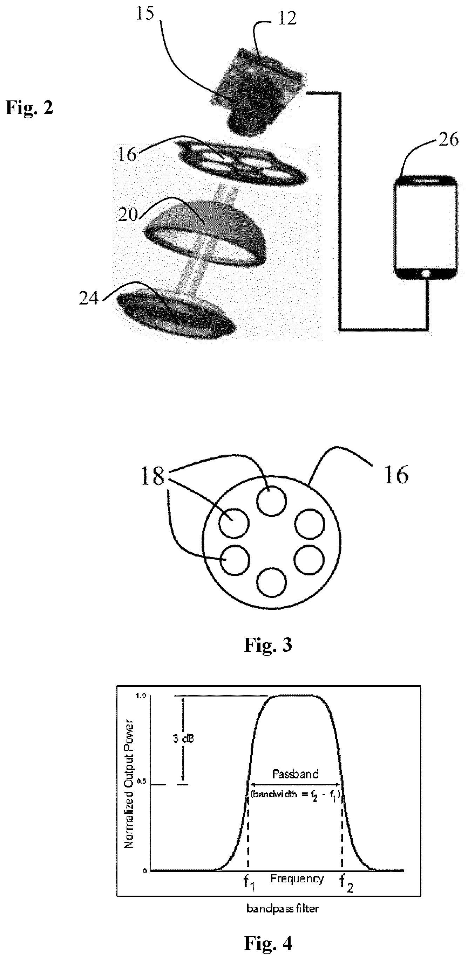

En se référant aux figures 1 à 4, un système **10** d'identification de microorganisme ayant poussées sous forme de colonie comporte :
- un dispositif d'acquisition d'image **12** comprenant un capteur d'image matriciel **14,** un objectif **15,** ainsi qu'un circuit de traitement recevant les signaux du capteur **14** et les configurant pour une transmission, par exemple au travers d'une connexion de type USB-C ou bluetooth, à une unité de traitement. Le capteur **14** est par exemple un capteur CMOS noir et blanc de 5 millions de pixels commercialisé par la société E-Con Systems, Inde sous la référence See3CAM_CU55M, pilotable depuis une application smartphone connecté par USB-C ;
- une roue à filtres **16,** comprenant un ensemble de filtres passe bande **18,** d'un nombre inférieur à 10. La roue est configurée par rapport au dispositif **12** pour être tournée par l'utilisateur de manière à présenter de manière séquentielle chacun des filtres **18** devant l'objectif **15.** En se référant à la figure 4, la largeur d'un filtre, définie comme la largeur à mi-hauteur de la réponse fréquentielle, est comprise entre 8nm et 50nm. Par exemple, les filtres sont des filtres passe-bande dichroïque d'une largeur de 10nm ;
- optionnellement un dispositif d'éclairage **20**, avantageusement sous la forme d'un dôme sous lequel une boite de Petri **22** comportant les colonies microbiennes à identifier;
- une unité de traitement d'image **26,** de préférence sous la forme d'un smarthphone ou d'une tablette, connectable au dispositif d'acquisition d'image 12, par exemple au moyen d'au câble USB-C

Le capteur d'image **14** du dispositif **12,** de technologie CMOS ou CCD, est un capteur "nu" en ce qu'il n'est revêtu ni de filtre infrarouge ni de matrice de Bayer comme cela est le cas des capteurs équipant les smarphones et tablettes à destination du grand public. En l'absence des filtres **18,** ce capteur monochrome est donc configuré pour intégrer le flux lumineux incident sur ses photosites sur l'ensemble de la plage [390-900]nm. Le montage optique, comprenant le capteur, le ou les objectifs avec leur(s) ouverture(s), ainsi que la distance du plan d'image du capteur à la boite de Pétri **22,** est configuré de manière à obtenir une image nette d'au moins 5 pixels, et de préférence d'au moins 10 pixels, d'une colonie microbienne ayant un diamètre compris entre 3 et 5mm, et ce quel que soit le filtre **18** présent devant les objectifs.

Le dispositif d'éclairage **20** comporte dans sa partie inférieure interne un anneau de LEDs **24** sélectionnées pour émettre un éclairage dans le visible et le proche infrarouge, notamment dans l'ensemble de la gamme [390-900]nm, par exemple des LEDs blanches commercialisées par la société Yujiled, Chine. Le dispositif **20** est conçu pour être un modeleur de lumière et comporte à cette fin sur sa paroi interne **26** une surface réfléchissante blanche ou argentée, par exemple un gris à 17%, de sorte à obtenir un éclairage uniforme évitant les phénomènes de spécularité sur les colonies ayant poussées sur la boite de Pétri **22.** Ce dispositif comprend également, dans le champ de vision du montage optique, une surface blanche afin de réaliser une correction de la température de couleur, ou correction du point blanc.

Le dispositif d'acquisition **12** est prévu en complément d'une unité de traitement **12** sous la forme d'un smartphone ou d'une tablette grand public. Ces derniers sont souvent la seule ressource informatique récente à disposition des personnels de santé et ont de multiples usages, tant professionnels que personnels. Ils sont donc plus sujets à des casses, pannes, ou des changements fréquents. En séparant ainsi de manière nette le dispositif d'acquisition d'image du smartphone en charge du traitement informatique des images, un double avantage est ainsi obtenu : a) la partie la plus "fragile" est remplaçable sans que le dispositif d'acquisition ne soit à changer; b) les smartphones étant produits en masse, leur coût est donc moindre qu'un dispositif qui serait produit spécifiquement pour mettre en oeuvre l'invention. Toutefois, l'invention couvre un smartphone ou une tablette dont le capteur et le système optique sont été spécifiquement conçus et produits pour mettre en oeuvre l'invention, le dispositif 12 étant alors omis et l'objectif du smartphone directement positionné devant la roue à filtres **16.**

Le dispositif d'éclairage **20,** bien qu'optionnel, est prévu pour faciliter grandement l'usage de l'invention. En effet, il permet d'obtenir un éclairage adéquat à l'intérieur du dôme, et donc sur la surface de la boite de Pétri, et ce quel que soit l'éclairage ambiant. Ce dispositif évite ainsi à l'utilisateur d'avoir à se préoccuper de cet aspect, et donc d'obtenir un diagnostic microbien quel que soient les conditions lumineuses ou les compétences de l'utilisateur en termes de réglages photographiques. De plus, il permet de positionner le dispositif **12** toujours à la même distance de la boite de Pétri, évitant ainsi à l'utilisateur d'avoir à se préoccuper également du réglage de le mise au point sur la boite de Pétri. La combinaison du dispositif d'acquisition d'image **12** et du dôme éclairant **20,** d'une hauteur combinée d'une dizaine de centimètre et d'un diamètre maximum du même ordre, permet donc à quiconque de prendre les images de la boite de Pétri **22,** sans avoir à suivre une longue formation, y compris les assistants des médecins, libérant ainsi du temps pour ces derniers. De manière avantageuse, la roue à filtres **16** est partie intégrante de la partie supérieure du dôme d'éclairage **20** de sorte à protéger au maximum cet élément qui est le plus fragile et le plus coûteux. Si le dôme est ici présenté rigide, il peut être prévu pliable pour augmenter sa transportabilité, par exemple du type des boites à lumière transportable connues dans le domaine de la photographie publicitaire ou culinaire.

Le traitement des images acquises est quant à lui mis en oeuvre de manière avantageuse sous la forme d'une application téléchargée sur le smartphone **26.** Si le paramétrage de ce traitement peut requérir des ressources informatiques importantes, notamment dans le cas d'un mode de réalisation à base d'apprentissage automatisé (ou "machine learning"), l'usage du traitement est cependant peu gourmand en ressources informatiques de sorte que la puissance de calcul des smartphones d'entrée de gamme à la date de dépôt de la présente demande est largement suffisante. On notera ainsi que le système selon l'invention, outre sa facilité d'usage, a un cout très modéré et peut donc être adopté à large échelle, en particulier dans les pays en voie de développement. Le traitement des images sera détaillé ci-après.

### B. Identification par un dispositif multi-spectral à l'aide d'un nombre limité de canaux spectraux

Il va à présent être un procédé d'identification de l'espèce microbienne formant colonies ayant poussées sur un milieux de culture COS (gélose Columbia au sang) parmi les espèces *Enterococcus faecalis (1), Escherichia coli (2), Klebsiella pneumoniae (3), Proteus mirabilis (4), Proteus vulgaris (5), Pseudomonas aeruginosa (6), Staphylococcus aureus (7), and Streptococcus agalactiae (8).* Les espèces ici listée sont choisies pour un diagnostic d'infection urinaire (ou "Urinary Tract Infection" - UTIs) et constituent plus de 90% de ces infections dans la majorité des hôpitaux. Le chiffre en parenthèse représente leur étiquette sur les figures.

Pour la conception de ce mode de réalisation, les inventeurs ont poursuivi trois objectifs de manière conjointe :
i. minimiser le nombre de filtres passe-bande qui constituent à ce jour la partie la plus onéreuse du système;
ii. obtenir une erreur de classification la plus faible possible, au maximum égale à 6%, ce seuil représentant la valeur au-delà duquel les microbiologistes et cliniciens considèrent le diagnostic comme scientifiquement trop peu fiable pour être utilisé en routine;
iii. et concevoir un algorithme d'identification qui est compréhensible par les utilisateurs, à la différence par exemple d'algorithmes à base d'apprentissage profond qui sont des "boites noires" pouvant former une barrière à l'adoption ou qui peuvent sujet à du surapprentissage. Les inventeurs ont cependant testé avec succès d'autres algorithmes de prédiction, comme par exemple des apprentissages automatisés à base de SVM ("Support Vector Machine") à noyau linéaire et radial, ou la PLS-DA.

Pour ce faire, les inventeurs se sont appuyés sur l'imagerie hyperspectrale. On sait que la gamme de longueurs d'onde 390-900nm contient l'information nécessaire à l'identification des espèces formant colonie avec une précision proche de 100%. Les inventeurs ont donc travaillé à la découverte de canaux spectraux spécifiques dans cette gamme les plus informatifs pour l'identification, tout en veillant à annuler les phénomènes de corrélation statistique entre canaux qui peuvent survenir dans ce type d'étude.

Pour ce faire, une base de données de spectre hyperspectraux de colonies des espèces est tout d'abord acquises. Notamment, plusieurs souches ont été collectés pour chaque espèce, puis chaque souche à faire l'objet d'un étalement sur trois boites de Pétri, mises en culture à 37°C pendant 24 heures, puis des images hypespectrales des boites sont acquises. Un total de 1745 colonies ayant poussées sur un milieu COS ont été analysées, constituées chacune d'une dizaine à une centaine de pixels. Les spectres issus de deux boites sont utilisés pour l'apprentissage d'un algorithme ci-après décrit et les spectres de la troisième boite sont utilisés pour le test de l'algorithme appris. Par exemple, l'acquisition des images des boites de Pétri dans la gamme [390-900]nm, et donc des colonies, est réalisée à l'aide d'une caméra hyperspectrale CMOS de 240 canaux d'une résolution spectrale de 2,1 nm. Ce système d'acquisition est par exemple celui décrit dans la demande de brevet WO 2019/122732. Le prétraitement des spectres acquis pour chaque pixel des colonies bactériennes est alors mis en oeuvre de manière à obtenir des spectres de réflectance corrigé des points blanc et noir, par exemple le pré-traitement décrit dans ce document. Les spectres prétraités, de réflectance, d'une colonie sont ensuite moyennés sur les pixels de cette colonie pour obtenir un seul spectre moyen par colonie.

Puis le spectre de réflectance moyen obtenu, d'une résolution initiale de 2,1nm, est sous-échantillonné dans le domaine des longueurs d'onde afin d'obtenir une résolution spectrale finale compatible avec les filtres passe-bandes du commerce dont la largeur est généralement comprise entre 8nm et 50 nm. Par exemple, une fenêtre glissante moyennant les intensités du spectre sur la largeur de la fenêtre est appliquée. A titre d'illustration, pour obtenir une résolution finale d'environ 10nm, une fenêtre de largeur égale à 4 canaux est appliquée, menant à une résolution de 4*2, 1nm. Le spectre de réflectance final est alors mémorisé dans la base de données.

Un total de 1745 colonies ayant poussées sur un milieu COS ont été analysées. Chaque souche bactérienne acquise pour l'étude a été étalée sous trois boites de Pétri, deux boites étant utilisées pour l'entrainement des algorithmes d'identification, la dernière étant utilisées pour tester les performances des algorithmes. Plusieurs algorithmes prédictifs ont été testés, notamment des prédiction de type Support Vector Machine, à noyau linéaire ou non linéaire, par exemple radiale, l'apprentissage étant réalisé selon la technique " 10-folds".

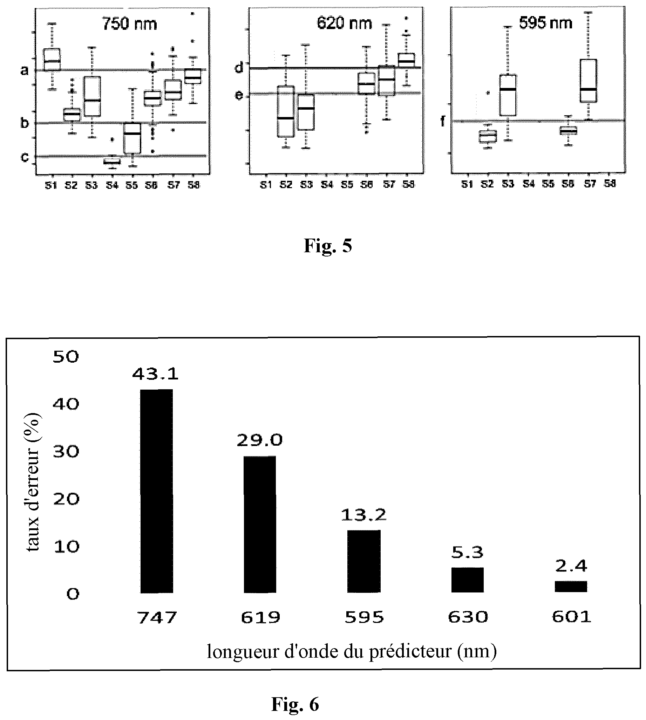

Un travail exploiratoire des descripteurs de ces algorithmes a été entrepris pour déterminer un nombre minimal de canaux permettant un taux d'erreur de classification inférieur à 10%, et de préférence inférieure ou égal à 6%. Notamment, une approche de type "feed forward" a été mise en oeuvre. Dans une première étape, le nombre de descripteur est posé égal à 1, et l'ensemble des canaux est parcouru pour déterminer celui qui est le plus prédictif. Ce canal est retenu et le nombre de descripteur est posé égal à 2, et les canaux restant sont parcourus pour déterminer le second canal le plus prédictif, et ainsi de suite. Comme illustré à la figure 5, le taux d'erreur chute rapidement en fonction du nombre de canaux sélectionnés, ce phénomène s'expliquant par le fait que les classes (étiquettes des espèces listées ci-dessus) sont séparables à la simple inspection visuelle de la réflectance desdits canaux (figure 4).

Les inventeurs ont ainsi découvert que 4 canaux seulement, d'une largeur comprise entre 8nm et 50nm et répartis dans les bandes [580-660]nm et [730-780]nm, permettent d'obtenir un taux d'erreur de classification inférieur à 6%. En particulier, les canaux comprenant la longueur d'onde proche de 750 nm (ici 747nm), de 620nm (ici 619nm) et 600nm (ici 595nm) permettent d'obtenir les performances visées.

Plus particulièrement, les inventeurs ont découvert que les trois canaux ayant les plus grandes valeurs prédictives de l'identification (en particulier en termes de taux d'erreur de classification ou en termes de spécificité) des espèces formant colonie sont compris dans les bandes [580-660]nm et [730-780]nm. La valeur prédictive d'un canal est par exemple déterminée par l'approche feed-forward telle que décrite précédemment. Elle est par exemple également déterminée par l'emploi du score PPS (pour "Power Predictive Score", décrit par exemple dans le document *"*RIP corrélation. Introducing the Prédictive Power Score", Florian Wetschoreckde, Toward Data Science, 2020). En particulier, en excluant ces bandes, les inventeurs ont découvert qu'il n'est pas possible d'obtenir une erreur de prédiction inférieure ou égale à 6%, au moins à l'aide des algorithmes de classification employés avec succès avec l'ensemble des canaux, par exemple les SVM à différents noyaux (linéaire et radial), la PLS-DA ou encore les algorithmes de régression logistic (e.g. du type LASSO). De manière avantageuse, un quatrième canal le plus contributeur à la performance de la prédiction est choisi dans la bande spectrale [580-660]nm ou [490-540]nm.

Dans un mode de réalisation privilégié, la prédiction des espèces, par exemple mise en oeuvre, sans y être limité, par le dispositif décrit en relation avec la figure 1, comprend l'acquisition d'images dans différents canaux spectraux, d'une largeur par exemple comprise entre 8 et 50nm, ces canaux étant compris uniquement dans les bandes [580-660]nm et [730-780]nm. Dans une variante, les canaux sont compris uniquement dans les bandes [580-660]nm, [730-780]nm et [490-540]nm.

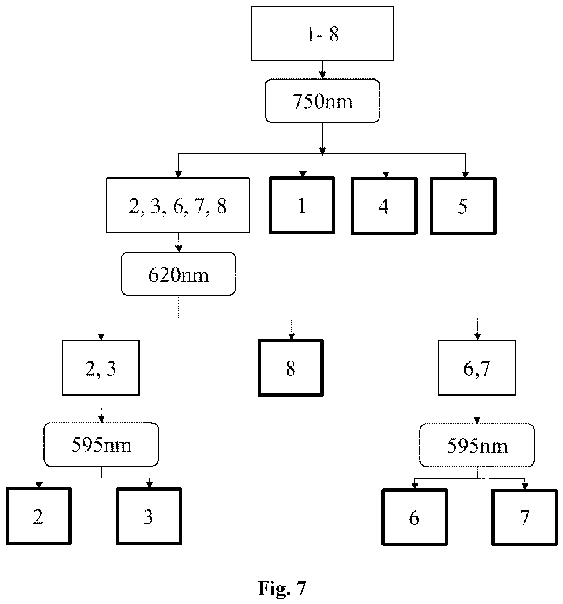

Dans une étape suivante, les inventeurs se sont attachés à concevoir un algorithme de prédiction d'interprétation simple en vue d'une adoption aisée de cette technologie. De manière avantageuse, comme illustré à la figure 7, un arbre décisionnel uniquement sur la base des quatre longueurs d'onde susvisées permet de prédire l'identité des espèces bactériennes formant colonie. Notamment, un canal comprenant la longueur d'onde 750nm permet d'identifier directement les classes 1, 4 et 5 dont les botploxs sont distinctes les unes des autres, ce qui permet de définir des gammes d'intensité les séparants. Les classes restantes (2, 3, 6, 7 et 8) sont ensuite séparées en fonction d'un canal comprenant la longueur d'onde 620nm, permettant d'identifier la classe 8, et un premier groupe comprenant les classe 2 et 3, et un second groupe comprenant les classes 6 et 7. Enfin un canal comprenant la longueur d'onde 595 nm permet de séparer les classes 2 et 3 d'une part et les classes 6 et 7 d'autre part. L'approche "feed foward", qui utilise des outils de machine learning pour identifier des canaux à fort poids de prédiction, permet donc d'identifer également les canaux pour lesquels les intensités lumineuses des espèces formant colonie sont séparables par un simple seuillage. On notera que dans ce mode de réalisation à base d'arbre décisionnel, aucune technique de machine learning n'est employées pour identifier les espèces, ce qui permet notamment de s'affranchir des bias d'apprentissage ainsi que de minimiser le risque de surapprentissage. Evidemment, l'invention couvre également les modèles d'apprentissage automatisé, en particulier des modèles de type Support Vector machine, par exemple à noyeau linéaire.

L'étude menée par les inventeurs sur la base d'une caméra hyperspectrale confirme donc la possibilité de mettre en oeuvre une approche multi-spectrale (i.e. basée sur un nombre de canaux limités) à base d'algorithmes de prédiction simples de compréhention (i.e. un arbre décisionnel) pour l'identification des espèces bactériennes sur boite de Pétri avec des performances acceptables en termes de diagnostice in vitro. Plus particulièrement, il est démontré que l'emploi d'une technologie multispectrale dont les performances d'acquisition de signal dans le canaux sélectionnés ayant la même qualité que la technologie hyperspectrale mène à une identification appropropiée.

### C. Industrialisation de l'identification multi-spectrale

Comme cela est parfois le cas, le passage d'un prototype de laboratoire basé sur une technologie spécifiquement conçue pour un objectif (une technologie hyperspectrale conçues pour une acquisition dans le visible et le proche infrarouge et utilisée en environnement contrôlé) à un dispositif commercial basée sur une autre technologie (ici une caméra monochromatique à base de CMOS ou CDD dont la réponse au-delà des 700nm est bruitée, associée à des filtres optiques du commerce et utilisée dans un environnement moins contrôlé) induit une dégradation des performances attendues. Ainsi, le choix des filtres correspondant aux canaux déterminés sur la base de la technologie hyperspectrale mène à une baisse sensible des performances, le taux d'erreur de classification pouvant être supérieur à 10%, voire 15%.

Selon l'invention, il s'agit donc de rétablir des performances de diagnostic in vitro, tout en respectant les contraintes d'usage, de robustesse, et de coût décrites ci-dessus. Pour ce faire, les inventeurs ont donc paramétré le dispositif multispectral selon l'invention en recherchant une synergie entre des caractéristiques techniques et des caractéristiques de worflow d'IVD microbiologique clinique:
i. Caractéristiques techniques multi-spectrales
   a. restriction de la gamme de longueurs d'ondes, notamment en diminuant la valeur du plus grand canal pour l'éloigner de la gamme de longueurs d'onde proche infrarouge où le capteur présente une réponse dégradée
   b. ajout d'un ou plusieurs canaux supplémentaires, et donc de filtres optiques passe-bande, au délà des 4 canaux déterminés via la technologie hyperspectrale. Au plus dix filtres sont utilisés. Au-delà, le système dans son entier, qui enchasse préférentiellement les filtres dans le dôme 20 pour leur protection, devient trop encombrant et/ou trop coûteux;
   c. optionnellement, le choix des filtres passe-bande pour régler une largueur de canal différente, notamment plus restreinte si cela permet d'augmenter les performances.
ii. Caractéristiques de workflow IVD clinique
   d. choix d'un panel d'espèces à identifier plus restreint si cela aide à améliorer la spécificité de la prédiction (c'est-à-dire la capacité à correctement prédire la bonne espèce parmis les échantillons comprenant effectivement au moins l'une des espèces du panel), la restriction étant cependant réalisée pour dégrader de manière minimale la prévalence de ce panel dans les infections, ici urinaire.
   e. le réglage de l'algorithme de prédiction de manière à prilégier la spécificité sur le taux de rejet (i.e. pourcentage d'échantillons pour lesquels aucun résultat d'identification n'est rendu).

Pour obtenir la synergie des caractéristiques a et b, l'approche "feed forward" est relancée sur la base de la technologie hyperspectrale, les caractéristiques d et e étant des hyperparamètres de l'algorithmes de prédiction. Concernant la caractéristique e, comme cela est connu en soi, les algorithmes à base de classification comprennent deux étapes: un premier étage renvoyant un ou plusieurs scores quantifiant une ou plusieurs distance de l'échantillon en cours de test aux différentes classes de l'algorithme et une second étage qui détermine une identité de classe en fonction des distances calculées en les comparants à un seuil prédéfini. La sélection de seuil permet de régler un compromis entre sensibilité ou spécificité par exemple. Si un arbre décisionnel est utilisé, la valeur des gammes d'intensité pour séparer les différentes classes et groupes de classe, mène également à un compromis entre spécificité et taux de rejet. Là encore, la spécificité est privilégiée.

Concernant la caractéristique d, il s'agit par exemple de paramétrer les caractéristiques a, b et e en fonction de différents panels choisis parmi une liste préatablie d'espèces, notamment les huit espèces décrites ci-dessus.

En procèdent de cette manière, les inventeurs ont conçus un premier mode de réalisation du dispositif de la figure 1 :
i. à base de 6 filtres optiques d'une largeur de 10nm, comprenant respectivement les longueurs d'onde 650nm 730nm, 670nm, 630nm, 471nm and 580nm;
ii. pour le panel d'espèces *Enterococcus faecalis (1), Escherichia coli(2), Klebsiella pneumoniae(3), Proteus mirabilis (4)* et *Streptococcus agalactiae (5)* qui comptent pour plus de 80% des inféctions urinaires
iii. avec une spécificité égale à 96% au détriment d'une sensibilité égale à 79%.

En poursuivant l'exploration synérgétique des caractéristiques a-d, les inventeurs ont conçu un second mode de réalisation du dispositif de la figure 1:
i. à base de 5 filtres optiques d'une largeur de 10nm, comprenant respectivement les longueurs d'onde 640nm, 660nm, 580nm, et 750nm,
ii. pour le panel d'espèces *Enterococcus faecalis (1), Escherichia coli(2), Klebsiella pneumoniae(3), Proteus mirabilis (4), Pseudomonas aeruginosa (5), Staphylococcus aureus (6),* et *Streptococcus agalactiae (7)* qui comptent pour plus de 80% des inféctions urinaires
iii. là encore avec une spécificité égale supérieure à 94%, au détriment d'une sensibilité proche de 80%.

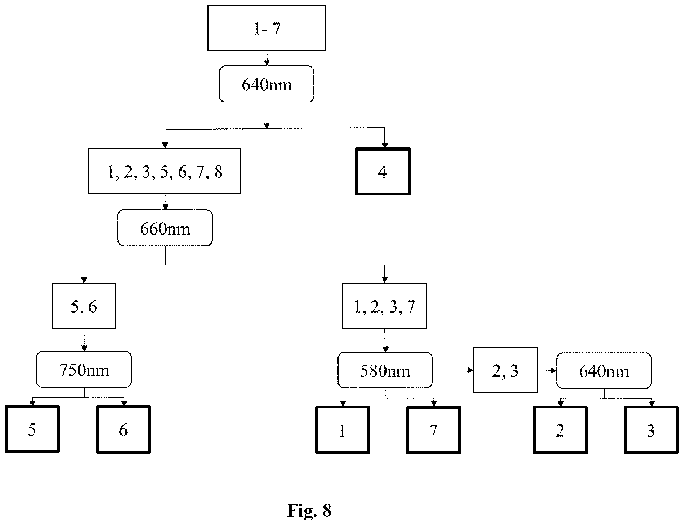

La figure 8 illustre un arbre décisionnel conçu par les inventeurs pour le mode de réalisation à 7 espèces, là encore dans un soucis d'adoption, les classes étant séparables directement par seuillage entre différent canaux par un choix particulier de la séquence de canaux à analyser.

Ainsi donc, le médecin peut avoir confiance dans le système lorsque ce dernier renvoie un résultat d'identification, la spécificité étant conforme aux obligations que doit respecter un dispositif certifié "diagnostic in vitro" (i.e. rendant scientifiquement un résultat que le médecin peut prendre pour "vérité" microbiologique, sans donc à avoir à procéder à des tests supplémentaire ou à interpréter ce dernier sur la base de son savoir-faire et/ou de la connaissance fine de l'écologie bactérienne auquel le patient est confronté).

Le taux de rejet d'échantillons positifs (i.e. échantillons pour lesquels on observe une pousse de colonie et une prédiction qui ne renvoie aucune identité) est cependant d'environ 20%, signifiant que pour un échantillon positif sur 5, le médecin ne dispose d'aucune information clinique. A contrario, 80% des échantillons positifs sont donc caractérisés de manière certaine, ce qui est à mettre au regard de certains cas où aucun échantillon n'est caractérisé du tout.

Dans un mode de réalisation priviligié, le taux de rejet des échantillons positifs est diminué par l'une au moins des caractéristiques supplémentaires suivantes, seule ou en combinaison:
e. un vote par boite: l'invention permet d'analyser plusieurs colonies, et non une seule comme cela est le cas des workflow cliniques classiques. En effet, il est d'usage en laboratoire clinique de prélever une seule colonie par boite, choix réalisé en fonction de l'expérience du technicien de laboratoire, pour réaliser les tests ultérieurs, comme par exemple un test d'identification à l'aide du spectromètre Vitek MS de la demanderesse ou un test d'antibiogramme à l'aide du Vitek 2 de la demanderesse. Grâce à l'invention, l'ensemble de la boite de Petri est imagée, et l'opérateur peut sélectionner, par exemple au moyen d'un écran et d'un pointeur contrôlés par l'application téléchargée sur le smartphone ou à l'aide d'un algoritme automatique de segmentation, plusieurs colonies. Chacune des colonie est alors analysée diminuant ainsi le risque de ne rendre aucune identification pour la boite. Un vote majoritaire sur l'espèce identifié est alors rendu à l'utilisateur, par exemple un vote majoritaire supérieur strictement à 50%, notamment supérieur ou égal à 60%.
f. l'ajout d'un filtre passe bande dans la gamme 415-440nm et la détermination du gram de la bactérie formant colonie par exemple comme cela est décrit dans la demande de brevet WO 2017/216190.
g. énumération des colonies. En disposant d'au moins une image complète de la boite de Pétri, il est possible de caractériser la charge bactérienne de l'échantillon, par exemple en mesurant sur l'image la surface des colonies (i.e.: les surfaces claires sur un medium de culture généralement sombre) et en calculant le ratio surface des colonies sur la surface de la boite. Dans le cas d'échantillon urinaire par exemple, stérile lorsque le patient n'est pas infecté, cela permet d'affirmer la présence d'une infection chez le patient.

Concernant la caractéristique f, la prédiction du Gram est réglée de manière à maximiser sa spécificité. De cette manière, si le dispositif ne rend aucune identification, il rend malgré tout un résultat sur le Gram d'au moins une espèce formant colonie sur laquelle le médecin peut se reposer pour déterminer une antibiothérapie empirique. Ainsi, le pourcentage d'échantillons ayant présenté une culture positive, n'ayant aucune caractérisation clinique, à savoir ni l'identité ni le Gram d'un pathogène compris dans un échantillon, est très faible, inférieur ou égal à 5%.

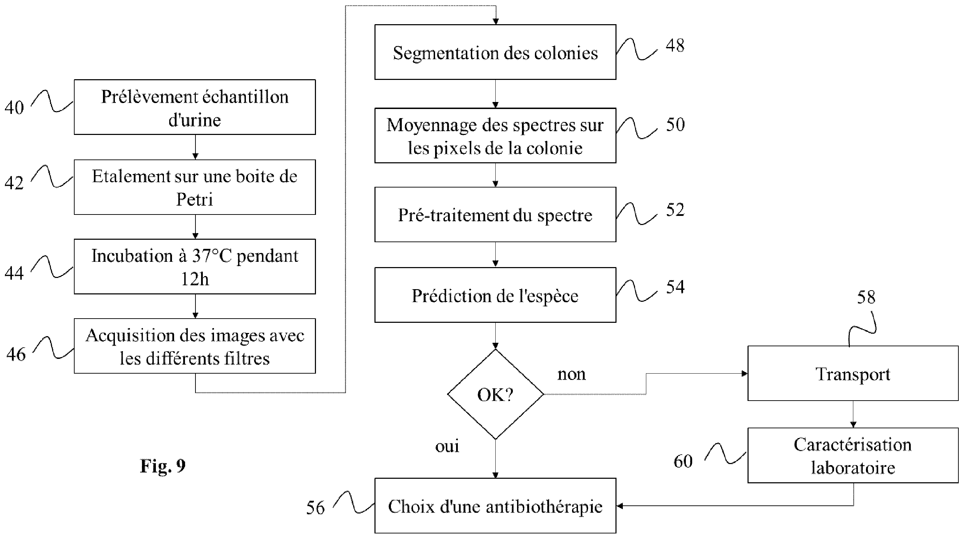

Un mode de réalisation d'un procédé de diagnostic et de thérapie anti-microbienne est par illustré à la figure **9** en relation avec des échantillons urinaires.

Le procédé débute en **40** par le prélèvement d'un échantillon urine chez un patient suspecté d'une infection urinaire, puis l'étallement en **42** de l'échantillon sur un milieu de culture compris dans une boite de Pétri (COS, TSS,...) puis la mise en culture de la boite, en **44,** à 37°C pendant au moins 12h. Un fois l'incubation terminée, en **46** un opérateur dispose la boite sous le dôme éclairant **20** du dispositif multi-spectral 10, et fait tourner la roue à filtre en prenant une image pour chaque filtre au moyen de l'application téléchargée sur le smartphone **26.** Une fois l'ensemble des images acquises, une ou plusieurs colonies sont sélectionnées en **48,** soit par l'opérateur à l'aide de l'écran du smartphone qui affiche l'image de la boite par l'un des filtres ou une image aggrégée, ou bien sélectionnée(s) à l'aide d'une algorithme de segmentation sélectionnant des objets 2D circulaires d'une manière connue en soi (par exemple par l'emploi de filtre d'image de type Hough). Pour chaque colonie sélectionnée, un moyennage de l'intensité des pixels de la colonie est réalisé pour chaque canal en **50,** menant ainsi à une valeur unique d'intentité par canal pour ladite colonie. Ce spectre multi-spectral moyen est alors optionnellement prétraité en **52,** par exemple pour régler ses points blanc et noir et/ou transformer son intensité en réflectance tel que décrit par exemple dans le document WO 2019/122732. Une identification de l'identité de la ou des espèces microbiennes formant la ou les colonies sélectionnées est alors mise en oeuvre en **54.** Si une identité est renvoyée par la prédiction, le médecin peut alorse, **56,** sélectionner la thérapie anti-microbienne en fonction de cette identifité et l'administrer au patient. Si aucune identification n'est renvoyée, la boite de Pétri, ou bien un aliquot de l'échantillon initial, est alors par exemple transporté, en **58,** vers un laboratoire doté d'instruments capables d'une caractérisation plus complète en **60,** caractérisation dont les résultats sont renvoyés par exemple au médecin sur son smartphone **26** qui choisit et administre la thérapie en fonction.

Dans une variante, lorsque le résultat de l'identification est négatif, le Gram est calculé si le dispositif **10** dispose du filtre nécessaire, d'une manière décrite ci-avant, ou tout autre valeur médicale utile au médecin (énumération, vote par boite, etc.).

Dans une variante de l'invention, il est décrit que c'est l'application téléchargée sur le smartphone qui réalise le stockage des images et la prédiction des espèces présentes dans l'échantillon. Dans une variante, lorsque le smartphone est connectable à un réseau de transmission de données (ADSL, fibre, par satellite...), ce stockage et cette prédiction sont réalisés sur un serveur distant, le smartphone étant utilisé pour transmettre les images audit serveur. De cette manière, la maintenance du smartphone et la mise à jour du logiciel de prédiction sont plus simples et plus robustes, le suivi des dossiers des patients peut être centralisé en temps réel, ou encore un technicien qualifié recevant de manière centralisée les images peut mettre son expertise au service de plusieurs médecins itinérants, réaliser un support en direct,etc.

Par unité de traitement informatique, on entend notamment tout matériel comprenant un ou plusieurs processeurs ou microprocesseurs associé(s) à des mémoires informatiques (cache, ram, rom,...) aptes à mémoriser des instructions pour la mise en oeuvre de calculs et traitements de données lorsqu'elles sont exécutées.

## Revendications

1. Procédé d'identification d'une souche microbienne sous forme de colonie, comportant :
A. l'acquisition d'un ensemble d'au moins trois canaux spectraux de la colonie compris dans le spectre visible et infrarouge;
B. la mise en oeuvre par ordinateur d'une prédiction de l'espèce en fonction des canaux spectraux acquis,
***caractérisé*** en ce les trois canaux dudit ensemble les plus contributeurs à la performance de la prédiction sont choisis dans :
- une première bande spectrale [730-780]nm;
- une deuxième bande spectrale [550-660]nm.

2. Procédé d'identification d'un microorganisme susceptible d'être contenu dans un échantillon biologique d'un type prédéfini, comprenant:
A. l'acquisition d'au moins une image numérique multispectrale d'au plus dix canaux d'au moins une colonie microbienne ayant poussée sur un milieu de culture suite à l'étallement de l'échantillon sur ledit milieu;
B. la prédiction, mise en oeuvre par informatique, de l'espèce microbienne formant ladite colonie parmi un panel prédéterminé d'espèces microbienne en fonction de la ou des images multispectrales numériques acquises;
***caractérisé* en ce que**:
- le panel d'espèces microbiennes, les filtres optiques et l'algorithme d'identification sont choisis de manière à ce que la spécificité de l'identification est supérieure ou égale à 90%, et de préférence supérieure ou égale à 94%;
- si le modèle de prédiction délivre l'identité d'une espèce
∘ alors ladite identité est confirmée sans mettre en oeuvre un autre procédé d'identification du microorganismes;
∘ sinon un autre procédé d'identification du microorganisme est mis en oeuvre.

3. Procédé selon la revendication 1 ou 2, ***caractérisé* en ce que** chaque canal spectral a une largueur comprise entre 8nm et 50nm.

4. Procédé selon la revendication 1, 2 ou 3, dans lequel ledit ensemble de canaux comprend un quatrième canal le plus contributeur à la performance de la prédiction compris dans la deuxième bande spectrale.

5. Procédé selon l'une des revendications précédente, dans lequel ledit ensemble de canaux comprend un quatrième canal le plus contributeur à la performance de la prédiction compris dans la bande spectrale [490-540]nm.

6. Procédé selon l'une des revendications précédentes, ***caractérisé* en ce que**
- l'acquisition est réalisée au moyen d'un capteur d'image monochromatique devant lequel sont positionnés de manière amovibles des filtres passe-bande correspondant aux canaux spectraux; et
- la largueur spectrale à mi-hauteur desdits filtres est comprise entre 8nm et 50nm.

7. Procédé selon l'une quelconque des revendications précédentes, ***caractérisé* en ce que** l'ensemble de canaux spectraux comprend au plus 10 canaux, et de préférence au plus 8 canaux spectraux.

8. Procédé selon l'une quelconque des revendications, ***caractérisé* en ce que** la prédiction de l'espèce de la souche bactérienne met en oeuvre un arbre décisionnel dans lequel chaque noeud comprend une prédiction réalisée en fonction d'un seul canal spectral de l'ensemble de canal spectral.

9. Procédé selon l'une quelconque des revendications précédentes, ***caractérisé* en ce que** l'espèce bactérienne est un uropathogène parmi les espèces *Escherichia coli, Enterococcus faecalis, Staphylococcus aureus, Proteus mirabilis, Proteus vulgaris, Klebsiella pneumoniae, Pseudomonas aeruginosa,* et *Streptococcus agalactiae.*

10. Dispositif d'identification d'une colonie microbienne parmi un panel prédéterminé d'espèces microbiennes responsables d'infections pathogéniques d'un type prédéterminé, notamment urinaire, comprenant:
A. une caméra numérique configurée pour acquérir une image numérique de ladite colonie;
B. et une unité de traitement informatique connectée à ladite caméra pour recevoir l'image numérique de la colonie;
***caractérisé en ce que*** la caméra numérique comprend:
A. 1. un capteur d'image monochromatique apte à capter une lumière incidente dans la gamme de longueurs d'onde [390-900] nm;
A.2.un objectif apte à former une image sur ledit capteur dans ladite gamme;
A.3.un ensemble d'au plus dix filtres passe-bande optiques centrés respectivement sur différentes longueurs d'onde de la gamme de longueurs d'onde [390-900] nm, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;
***et en ce que*** les trois canaux dudit ensemble les plus contributeurs à la performance de la prédiction sont choisis dans :
- une première bande spectrale [730-780]nm;
- une deuxième bande spectrale [550-660]nm

11. Dispositif d'identification d'une colonie microbienne parmi un panel prédéterminé d'espèces microbiennes responsables d'infections pathogéniques d'un type prédéterminé, notamment urinaire, comprenant:
A. une caméra numérique configurée pour acquérir une image numérique de ladite colonie;
B. et une unité de traitement informatique connectée à ladite caméra pour recevoir l'image numérique de la colonie;
***caractérisé en ce que*** la caméra numérique comprend:
A.1. un capteur d'image monochromatique apte à capter une lumière incidente dans la gamme de longueurs d'onde [390-900] nm;
A.2.un objectif apte à former une image sur ledit capteur dans ladite gamme;
A.3.un ensemble d'au plus dix filtres passe-bande optiques centrés respectivement sur différentes longueurs d'onde de la gamme de longueurs d'onde [390-900] nm, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;
***en ce que*** l'unité de traitement informatique est configurée pour mettre en oeuvre un algorithme d'identification de l'espèce formant la colonie en fonction des images numériques,
***en ce que*** le panel d'espèces microbiennes correspond à une prévalence d'au moins 70% des infections pathologiques, et de préférence supérieure ou égale à 80%,
***et en ce que*** le panel d'espèces microbiennes, les filtres optiques et l'algorithme d'identification sont choisis de manière à ce que la spécificité de l'identification est supérieure ou égale à 90%, et de préférence supérieure ou égale à 94%.

12. Dispositif de caractérisation d'une colonie microbienne, comprenant:
A. une caméra numérique configurée pour acquérir une image numérique de ladite colonie;
B. et une unité de traitement informatique connectée à ladite caméra pour recevoir l'image numérique de la colonie;
***caractérisé en ce que*** la caméra numérique comprend:
A. 1. un capteur d'image monochromatique apte à capter une lumière incidente dans la gamme de longueurs d'onde [390-900] nm;
A.2.un objectif apte à former une image sur ledit capteur dans ladite gamme;
A.3.un ensemble d'au plus dix filtres passe-bande optiques, chacun ayant une largeur de bande comprise dans la gamme [8-50]nm, chacun desdits filtres étant configuré pour être placé de manière amovible devant l'objectif de manière à acquiérir une image numérique multispectrale de la colonie microbienne;
***en ce que*** l'ensemble d'au plus 10 filtres comprend:
A.3. 1.un premier filtre centré sur une longueur d'onde de la gamme [415-440]nm;
A.3.2.des seconds filtres centrés respectivement sur différentes longueurs d'onde de la gamme de longueurs d'onde [390-900] nm
***et en ce que*** l'unité de traitement informatique est configurée pour identifier:
B.1.le gram de l'espèce formant la colonie en fonction de l'image numérique de ladite colonie acquise au moyen du premier filtre;
B.2. l'identité de l'espèce formant la colonie en fonction des images numériques acquises à l'aide des seconds filtres.

13. Dispositif selon l'une des revendications 10 à 12, **caractérisé en ce qu'**il est configuré pour mettre en oeuvre un procédé selon l'une des revendications 1 à 10.
